(19)

Europäisches
Patentamt
European
Patent Office
Office européen
des brevets

(11) **EP 2 422 784 A1**

(12) **EUROPEAN PATENT APPLICATION**

(43) Date of publication:
 **29.02.2012 Bulletin 2012/09**

(51) Int Cl.:
 *A61K 31/513* (2006.01)  *A61K 31/216* (2006.01)
 *A61P 35/00* (2006.01)  *A61K 31/353* (2006.01)

(21) Application number: **10173256.8**

(22) Date of filing: **18.08.2010**

(84) Designated Contracting States:
 **AL AT BE BG CH CY CZ DE DK EE ES FI FR GB GR HR HU IE IS IT LI LT LU LV MC MK MT NL NO PL PT RO SE SI SK SM TR**
 Designated Extension States:
 **BA ME RS**

(71) Applicant: **Grindeks, a joint stock company**
 **1057 Riga (LV)**

(72) Inventors:
 • **Kalvins, Ivars**
  **1006 Riga (LV)**

• **Stonans, Ilmars**
 **1057 Riga (LV)**
• **Sestakova, Irina**
 **1006 Riga (LV)**
• **Domracova, Ilona**
 **1006 Riga (LV)**
• **Jascenko, Elina**
 **1006 Riga (LV)**
• **Bridane, Veronika**
 **1006 Riga (LV)**
• **Kanepe, Iveta**
 **1006 Riga (LV)**

(54) **Composition of TEGAFUR, caffeic acid phenethyl ester (CAPE) and either catechin, kaempherol, myricetin or luteolin for potentiating antitumour effect and for treating tumours**

(57) The present invention provides a composition of one or more natural flavonoid derivatives, caffeic acid phenethyl ester (CAPE) and a cancer chemotherapeutic agent TEGAFUR. In one embodiment of the present invention, a combination of natural flavonoid derivatives, caffeic acid phenethyl ester (CAPE) and TEGAFUR can be used in the treatment of tumours, preferably malignant tumours.

EP 2 422 784 A1

**Description**

Technical Field

The present invention relates to a novel composition of TEGAFUR, caffeic acid phenethyl ester and natural flavonoid derivate for potentiating antitumour effect in the treatment tumours; in particular, it relates to the unexpected synergism of a combination of Tegafur, caffeic acid phenethyl ester and a natural flavonoid derivate in the treatment of tumour; and to processes for preparing the composition and its use for chemotherapy of tumours, especially malignant tumours.

Background Art

Cancer is the uncontrolled growth and spread of cells that may affect almost any tissue of the body. Lung, colorectal and stomach cancer are among the five most common cancers in the world both men and women. Among men, lung and stomach cancer are the most common cancers worldwide. For women, the most common cancers are breast and cervical cancer.
More than 11 million people are diagnosed with cancer every year. It is estimated that there will be 16 million new cases every year by 2020. Cancer causes 7 million deaths every year - or 12.5% of deaths worldwide.
Therefore, a great number of investigations, developments and research works have been carried out to find anticancer agents, which can be used in chemotherapy of malignant tumours. There are known various anticancer agents and results of cancer treatment improve with every year, nevertheless the amount of effective dose of anticancer agent for treatment is still high.
One of such anticancer agents is TEGAFUR. TEGAFUR, i.e. 1-(2-tetrahydrofuryl)-5-fluorouracil, is disclosed in GB 1 168 391 (INST ORGANOCHESKOGO SINTEZA, published 22.10.1969) as a prodrug of 5-FU, i.e. 5-fluorouracil, and proved active in the management of metastatic colorectal cancer. TEGAFUR is metabolized to 5-fluorouracil (5-FU) in vivo, predominantly in the liver, and has been reported to be less toxic and to have a higher therapeutic index CAO than 5-fluorouracil prodrug. It plays a role in anabolic and catabolic pathway modulation in therapy of colorectal cancer (see "Cancer Research" 1995, vol. 1, p.839-845).
US 4 328 229 (TAIHO PHARMACEUTICAL, published 04.05.1982) discloses an anti-cancer composition, containing 1-(2-tetrahydrofuryl)-5-fluorouracil (TEGAFUR) and uracil. The composition is used for delivery of 5-fluorouracil to a tumour which is sensitive to 5-fluorouracil in a warm-blooded animal. It is disclosed therein that the composition can be coadministered in a variety of dosage forms including an oral dosage form.
Caffeic acid phenethyl ester (CAPE) is a phenolic compound contained in propolis, which is the generic name of a resinous product derived from the bark of conifer trees and carried by honeybees to their hives (see CHIAO, C. "Apoptosis and altered redox state induced by caffeic acid phenethyl ester (CAPE) in transformed rat fibrolast cells", published in "Cancer Research" 1995, vol.55, no.3576, p.3583).
Caffeic acid phenethyl ester (CAPE) has been shown to display increased toxicity toward various oncogene-transformed cell lines in comparison with their untransformed conterparts (see Guarini L, Su ZZ, Zucker S, Lin J, Grunberger D, Fisher PB "Growth inhibition and modulation of antigenic phenotype in human melanoma and glioblastoma multiforme cells by caffeic acid phenethyl ester (CAPE)", published in "Division of Pediatric Hematology/ Oncology" 1991, 4:231-242).
The biological activities of propolis and CAPE have been studied, and it has been shown that CAPE also has an anti-tumoral activity. Various investigators have demonstrated that CAPE also has an anti-inflammatory properties both in vitro and in vivo (see MIRZOEVA, O., "The effect of propolis and its components on eicosanoid production during the inflammatory response", published in "Prostaglandins Leukotienes Essent Fatty Acids", p.441-449, and ORBAN, Z., "Caffeic acid phenethyl ester induces leukocyte apoptosis, modulates nuclear factor-kappa B and suppresses acute inflammation", published in "Neuroimmunomodul" 2000, vol.7, p.99-105). CAPE has also been proposed as a specific inhibitor of the Transcription Factors Nuclear Factor {kappa} B, which may account for its anti-inflammatory actions. Specifically, in a human histiocytic cell line, CAPE only inhibited NF-K B, as opposed to other transcription factors, such as API, TFIID and Oct-1. In this regard, it has been proposed to use CAPE directly for inhibiting the interaction of NF-K13 with DNA (see NATARAJAN, K.et.al., "Caffeic acid phenethyl ester is a potent and specific inhibitor of activation of nuclear transcription factor NF-kappa B, published in "Immunology", 1996, vol. 93, p.9090-9095). CAPE also clearly induces apoptosis in various cell types.
US 5008441 (UNIV COLUMBIA, published 16.04.1991) discloses a method for producing caffeic acid phenethyl ester (CAPE) and a method which substantially inhibits the growth of transformed cells without substantially inhibiting the growth of normal cells. This process comprises treating a population of cells with an effective inhibiting amount of caffeic acid phenethyl ester (CAPE) so as to substantially inhibit the growth of the transformed cells. Transformed cells are human breast carcinoma cells, human melanoma cells (SK-MEL-28 or SK-MEKL-1 70) or colon or renal carcinoma cells. In MASAHIKO, W., et al., "Caffeic Acid Phenethyl Ester Induces Apoptosis by Inhibition of NF (kappa) B and Activation of Fas in Human Breast Cancer MCF-7 Cells", published in "Journal of Biological Chemistry" 2004, vol.279, no.7, p.6017-6026, caffeic acid phenethyl ester, is described as a flavonoid derivative, which is a biologically active ingredient of honeybee propolis and strongly inhibits nuclear transcription factor NF-{kappa} B activation. Furthermore, it was demonstrated that apoptosis is induced by CAPE in various cancer cell lines - e.g. human breast cancer MCF-7 cells. It was found in by TAKEMA, N., et al., in "Selective antiproliferative activity of caffeic acid phenethyl ester analogues on highly liver-metastatic murine colon 26-L5 carcinoma cell line", published in "Bioorganic&Medicinal Chemistry" 2002, vol. 10, no.10, p. 3351-3359, that CAPE possesses selective antiproliferative activity toward a highly liver-metastatic murine colon 26-L5 carcinoma cell line.

WO 03/090 681 (RES DEV FOUNDATION, published 11.06.2003) discloses synergistic effects of nuclear transcription factor NF-{kappa} B inhibitors and anti-neoplastic agents in the treatment of cancer. As an NF-K B inhibitor caffeic acid phenethyl ester (CAPE) and as an anti-neoplastic agent 5-fluorouracil (5-Fu) is mentioned. The human breast cancer cell lines MDA-MB 435 and MCF-7 were treated with a nuclear transcription factor NF-{kappa} B inhibitor and an anti-neoplastic agent.

WO 2005/105 086 (TAIHO PHARMACEUTICAL CO LTD, published 11.10.2005) discloses a combination of the three drugs TEGAFUR, 2,4-dihydroxy-5-chloropyridine and oxonic acid or a pharmaceutical accteptable salt thereof in a mole ratio of 1:0.4:1, which is available as capsules. This anti-tumour agent reduces the gastrointestinal toxicity and improves the tolerability of 5-Fu. The main effect of the anti-tumour agent achieved by its 2,4-dihydroxy-5-chloropyridine component, which exhibits a 5-Fu degradation inhibitory effect being about 180 times greater than that of uracil.

Flavonoids are polyphenolic secondary metabolites widely dispersed throughout the plant kingdom and found in substantial levels in commonly consumed fruits, vegetables and beverages. The flavonoids, which are benzo-{gamma}-pyrone derivates with A, B and C rings, are categorized as: flavonols, flavones, flavan-3-ols, isoflavones, flavanones and anthocyanidins. They have been shown to possess a variety of biological activities at non-toxic concentrations in organisms. The role of dietary flavonoids in cancer prevention is widely discussed. Compelling data from laboratory studies, epidemiological investigations, and human clinical trials indicate that flavonoids have important effect on cancer chemoprevention and chemotherapy. Many mechanisms of action have been identifies, including carcinogen inactivation, antiproliferation, cell cycle arrest, induction of apoptosis and differentiation, inhibition of angiogenesis, antioxidation and reversal of multidrug resistance or a combination of these mechanisms. Based on these results, flavonoids may be promising anticancer agents, published in "Medical Research Review" 2003, vol.23, no. 4, p. 519-534.

Myricetin is a naturally-occurring flavonoid found in many grapes, berries, fruits, vegetables, herbs, as well as other plants but the most myricetin has been found in cranberries.

Myricetin have antioxidant properties, antibacterial and antiviral activity and may have anti-inflammatory activity. In test tube studies, myricetin has shown anti-tumour.

Kaempferol is a flavone-ring-containing plant pigment found in many angiosperms (flowering plants). Kaempferol is a natural flavonoid which has been isolated from tea, broccoli, Delphinium, Witch-hazel, grapefruit, and other plant sources. Kaemphferol consumption in tea and broccoli has been associated with reduced risk of heart disease.

Catechins are polyphenolic antioxidant plant metabolites, they are specially flavonoids called flavan-3-ols.

Catechins have potent antioxidant and cancer chemopreventive properties. These compounds are found in a variety of plants and are present in particularly high amounts in tea leaves, published in "Cancer research" 2001, no. 61, p.118-125.

Green tea and, to a lesser extent, black tea are rich sources of catechins. Studies by Japanese researchers have shown that catechins can prevent free radical damage of cholesterol and lower the risk of heart disease and cancer, published in http://www.thenutritionreporter.com/power-_of_flavonoids.htm 103/07/2007

High levels of monomeric (+)-catechin are found in the skin and seeds of fruits such as apples and grapes. Red wine and chocolate also are significant sources of (+)-catechin. In keeping with the ability of phenolic antioxidants to induce the expression of phase II detoxifying enzymes in mammalian cells, (+)-catechins produces antimutagenic effects, published in "Cancer research" 2001, no. 61, p.118-125.

Disclosure of Invention

Technical problem

In malignant tumour treatment different kind of pharmaceutical compositions are used, including pharmaceutical compositions of TEGAFUR, but just only partial remission of malignant tumour was obtained.

As well as composition which is disclosed in WO 2005105086 (TAIHO PHARMACEUTICAL CO LTD) 11.10.2005 said that it reducing the gastrointestinal toxicity of TEGAFUR and improving tolerability of 5-Fu, but still only partial remission of tumour is developed.

The further aim of present invention is to developed effective pharmaceutical composition that can be used in malignant tumour treatment for achieving increased remission of tumours.

Technical solution

The present invention is directed to a combination for treating a tumour, especially a malignant tumour in an individual who need such treatment, comprising the administration to said individual of a pharmacologically effective dose of derivatives of natural compounds and the cancer chemotherapeutic agent TEGAFUR.

While attempting to develop a pharmaceutical composition for malignant tumour treatment having an essentially lower toxicity, we unexpectedly found that doses adequate to the therapeutic ones used in the clinical use of pharmaceutical compositions containing TEGAFUR [1-(2-tetrahydrofuryl)-5-fluorouracil], a drug for treating a malignant tumour, the co-administration or combined use in pharmaceutical compositions of a compound of the caffeic acid phenethyl ester (CAPE) and natural derivatives of flavonoid.

By using a composition of Tegafur, caffeic acid phenethyl ester (CAPE) and one natural flavonoid derivative the following advantageous effects are attained:

- Tegafur potentiating anti-tumour effect of caffeic acid phenethyl ester and natural flavonoid derivate in the treatment of tumours;
- Combination of Tegafur, CAPE and a natural flavonoid derivate showed remarkable synergism between the components in the treatment of tumour;
- The use of a combination of Tegafur, CAPE and one of natural flavonoid derivative, preferably (+)-Catechin, Myricetin, Kaempferol, allows efficacy increase growth inhibition of S-180 ascites tumour.

There is no particular restriction on the unit dosage from which can be adopted for the anti-tumour composition of the invention in the treatment of malignant tumours in mammals. The unit dosage form is selected according to the purpose of treatment. Examples thereof are oral dosage form such as tablets, coated tablets, pills, powders, granules, capsules, solutions, suspensions, emulsions, etc. and parenteral dosage forms such as suppositories, ointments, plasters, etc. These dosage dorms can be manufactured by convention pharmaceutical procedures known in this field. As the carrier for shaping into the form of tablets, there can be employed various excipients such as lactose, sucrose, sodium, glucose, urea, starch, calcium carbonate, kaolin, crystalline cellulose, silicic acid, etc.; binders such as simple syrup, glucose solution, starch solution, gelatine solution, carboxymethylcellulose, shellac, methylcellulose, potassium phosphate, polyvinylpyrrolidone, etc,; disintegrators such as dry starch, sodium alginate, agar powder, laminaran powder, sodium hydrogen carbonate, calcium carbonate, polyoxyethylene-sorbitan fatty acid esters, sodium lauryl sulphate, stearic acidmonoglyceride, starch, lactose, etc.; antidisintegrators such as sucrose, stearic acid, cacao butter, hydrogenated oil etc.; absorption promotors such as quaternary ammonium bases, sodium lauryl sulphate, etc.; humectants such as glycerol, starch, etc.; adsorbents such as starch, lactose, kaolin, bentonite, colloidal silicic acid, etc.; and lubricants such as purified talc, stearic acid salts. Boric acid powder, polyethylene glycol, etc. Where necessary, the tablets may be in the form of coated tablets such as sugar-coated tablets, gelat-coated tablets, enteric-coated tablets, film-coated tablets, or double or multi-layer tablets, etc.

The carrier for shaping into the form of pills includes, for example, various excipients such as glucose, lactose, starch, cacao butter, hardened vegetable oil, kaolin, talc, etc.; and disintegrators such as laminarian, agar, etc. The carrier for shaping into the form of suppositories includes, for example, polyethylene glycol, cacao butter, higher alcohols, esters of higher alcohols, gelatine, semi-synthetic glycerides, etc.

Capsules are manufactured by mixing the derivatives of natural compounds with TEGAFUR, with any of the carriers mentioned above and encapsulating the mixture in hard gelatine capsule, soft capsule or other capsules.

For manufacturing in the form of pastes, creams and gels, there is employed a diluents such as, for example, white petrolatum, paraffin, glycerol, cellulose derivatives, polyethylene glycols, silicone, bentonite, etc.

Best Mode for Carrying Out the Invention

The present invention in the following will be described in more detail with reference to pharmacological tests and examples illustrating the preparation of the anti-tumour effect-potentiating compositions of the invention comprising caffeic acid phenethyl ester and natural derivatives of flavonoid and TEGAFUR.

PHARMACOLOGICAL TEST

Pharmacological test was carried out to demonstrate a substantial antimetastatic effect of composition of TEGAFUR and caffeic acid phenethyl ester and natural derivatives of flavonoids

Experimental example

Experiments in vivo were carried out on mice sarcoma S-180 ascites form.

The anti-tumour activity of the pharmaceutical composition of TEGAFUR, caffeic acid phenethyl ester and one of natural flavonoid derivatives was determined by introducing them into ICR mice of initial weight 20-23 g during a fortnight. Throughout the experiment, the mice were kept under standard laboratory conditions at a temperature of $22\pm2$ °C, relative humidity 55 $\pm$15%, ventilation - 15-20 changes of air amount/hour and a 12 hours light/darkness cycle, and were fed with standard laboratory animal food.

Mice sarcoma S-180 transplanted intraperitoneally to ICR mice in amount 5 x $10^6$ cell/mouse.

Then, 24 hours later, orally administration of compounds was started once a day at the 1st, 3rd, 5th, 7th, 9th, 11th and 13th days.

The compound efficacy was expressed as the percentage of growth inhibition of S-180 ascites tumour, calculated using the equation:

$$\text{Growth inhibition of S-180 ascites tumour}(\%) = 100 - \left( \frac{T}{C} \times 100 \right)$$

wherein C - mean weight (g) of the control group and T - mean weight (g) of the experimental group.

The results of Table 1 show the growth inhibition of S-180 ascites tumour at 13th day after the transplantation of tumour in mice.

Table 1

| Nr. | Drug | Dosage (mg/kg) | Growth inhibition of S-180 ascites tumour, % |
|---|---|---|---|
| 1 | TEGAFUR | 25 | 9 |
| 2 | CAPE | 12.5 | -1 |
| 3 | Catechin | 300 | 6 |
| 4 | TEGAFUR+CAPE+ Catechin | 25+12.5+300 | 21 |
| 5 | TEGAFUR | 25 | 9 |
| 6 | CAPE | 50 | 24 |
| 7 | Kaempferol | 100 | -9 |
| 8 | TEGAFUR+CAPE+ Kaempferol | 25+50+100 | 58 |
| 9 | TEGAFUR | 25 | 9 |
| 10 | CAPE | 50 | 24 |
| 11 | Myricetin | 50 | -9 |
| 12 | TEGAFUR+CAPE+ Myricetin | 25+50+50 | 61 |

As it is shown in Table 1 combinations of Tegafur, CAPE and one of natural flavonoid derivative showed remarkable synergism between the components and allows efficacy increase growth inhibition of S-180 ascites tumour.

Formulation Example 1: Granules

A possible pharmaceutical composition for oral use exemplifying but not exhausting the present invention is the following formulation of granules manufacture:

| | Example1 | Example2 | Example3 |
|---|---|---|---|
| Tegafur | 25 mg | 25 mg | 25 mg |
| CAPE | 27 mg | 50 mg | 50 mg |
| (+)-Catechin | 300 mg | 0 | 0 |
| Myricetin | 0 | 0 | 50 mg |
| Kaempferol | 0 | 100 mg | 0 |

(continued)

|  | Example1 | Example2 | Example3 |
|---|---|---|---|
| Lactose | 400 mg | 400 mg | 400 mg |
| Corn starch | 400 mg | 400 mg | 400 mg |
| Hydroxymethylcellulose | 13 mg | 10 mg | 10 mg |
| Total | 1162 mg | 985 mg | 935 mg |

Formulation Example 2: Tablets

A possible pharmaceutical composition for oral use exemplifying but not exhausting the present invention is the following formulation of tablets production:

|  | Example1 | Example2 | Example3 |
|---|---|---|---|
| Tegafur | 20 mg | 20 mg | 20 mg |
| CAPE | 20 mg | 40 mg | 30 mg |
| (+)-Catechin | 40 mg | 0 | 0 |
| Myricetin | 0 | 0 | 30 mg |
| Kaempferol | 0 | 40 mg | 0 |
| Lactose | 100mg | 100 mg | 100 mg |
| Crystalline cellulose | 50 mg | 30 mg | 50 mg |
| Magnesium stearate | 10 mg | 10 mg | 10 mg |
| Talc | 10 mg | 10 mg | 10 mg |
| Total | 250 mg | 250 mg | 250 mg |

Formulation Example 3: Capsules

A possible pharmaceutical composition for oral use exemplifying but not exhausting the present invention is the following formulation of capsules production:

|  | Example1 | Example2 | Example3 |
|---|---|---|---|
| Tegafur | 50 mg | 25 mg | 50 mg |
| CAPE | 50 mg | 50 mg | 100 mg |
| (+)-Catechin | 100 mg | 0 | 0 |
| Myricetin | 0 | 0 | 100 mg |
| Kaempferol | 0 | 100 mg | 0 |
| Lactose | 50 mg | 50 mg | 50 mg |
| Talc | 50 mg | 50 mg | 50 mg |
| Ca stearate | 6 mg | 7 mg | 7 mg |
| Aerosil | 4 mg | 3 mg | 3 mg |
| Total | 310 mg | 285 mg | 360 mg |

Formulation Example 4: Suppository

A possible pharmaceutical composition for internal use exemplifying but not exhausting the present invention is the following formulation of suppository production:

|  | Example 1 | Example 2 | Example 3 |
|---|---|---|---|
| Tegafur | 100 mg | 50 mg | 100 mg |
| CAPE | 100 mg | 100 mg | 200 mg |
| (+)-Catechin | 200 mg | 0 | 0 |
| Myrcetin | 0 | 0 | 200 mg |
| Kaempferol | 0 | 200 mg | 0 |
| Witepsol W-35 | 800 mg | 850 mg | 700mg |
| Total | 1200 mg | 1200 mg | 1200 mg |

Formulation Example 5: Suspensions

A possible pharmaceutical composition for topical use exemplifying but not exhausting the present invention is the following formulation of suspension production:

|  | Example 1 | Example 2 | Example 3 |
|---|---|---|---|
| Tegafur | 50 mg | 50 mg | 100 mg |
| CAPE | 55 mg | 100 mg | 200 mg |
| (+)-Catechin | 300 mg | 0 | 0 |
| Myricetin | 0 | 0 | 200 mg |
| Kaempferol | 0 | 200 mg | 0 |
| Glycerin | 100 mg | 100 mg | 100 mg |
| Disstilled water | 155 mg | 200 mg | 100 mg |
| Total | 660 mg | 650 mg | 700 mg |

Industrial Application

According to the invention, the pharmaceutical composition for treating malignant tumour which contains Tegafur, Caffeic acid phenethyl ester and one of natural flavonoid derivative, preferably (+)-Catechin, Kaempferol, Myricetin in clinically efficacious amount might be manufactured in a standard pharmaceutical plant.

**Claims**

1. A pharmaceutical composition comprising Tegafur [1-(2-tetrahydrofuryl)-5-fluorouracil], caffeic acid phenethyl ester (CAPE) and one natural flavonoid derivative, selected from the group of Catechin, Kaempferol, Myricetin or Luteolin.

2. A pharmaceutical composition according to claim 1, wherein natural flavonoid derivative is Catechin.

3. A pharmaceutical composition according to claim 1, wherein natural flavonoid derivative is Kaempferol.

4. A pharmaceutical composition according to claim 1, wherein natural flavonoid derivative is Myricetin.

5. A pharmaceutical composition according to claim 1, wherein natural flavonoid derivative is Luteolin.

6. A pharmaceutical composition according to claim 1, wherein preferable ratio of the active ingredients Tegafur, caffeic acid phenethyl ester (CAPE) and one natural flavonoid derivative, selected from the group of Catechin, Kaempferol, Myricetin or Luteolin, is from 1:100:100 to 100:1:1.

7. A pharmaceutical composition according to claim 1, wherein preferable ratio of the active ingredients Tegafur, caffeic acid phenethyl ester (CAPE) and one natural flavonoid derivative, selected from the group of Catechin, Kaempferol, Myricetin or Luteolin, is from 1:20:20 to 20:20:1.

8. A pharmaceutical composition according to claim 1, wherein preferable ratio of the active ingredients Tegafur, caffeic acid phenethyl ester (CAPE) and one natural flavonoid derivative, selected from the group of Catechin, Kaempferol, Myricetin or Luteolin, is from 1:2:2 to 2:1:1.

9. Use of pharmaceutical composition of any preceding claim as a medicament.

10. A pharmaceutical composition according to claims 1-8 for use in the treatment of a tumour.

11. Use of a pharmaceutical composition according to claims 1-8 for the manufacture of a medicament for administration simultaneously, sequentially or separately for treatment and/or prevention of tumour.

Europäisches
Patentamt

European
Patent Office

Office européen
des brevets

**EUROPEAN SEARCH REPORT**

Application Number

EP 10 17 3256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/031766 A1 (GRINDEKS A JOINT STOCK COMPANY [LV]; STONANS ILMARS [LV]; KALVINS IVAR) 25 March 2010 (2010-03-25) * claims * | 1-11 | INV. A61K31/513 A61K31/216 A61P35/00 A61K31/353 |
| Y | SUZUKI I ET AL: "Antitumor and anticytopenic effects of aqueous extracts of propolis in combination with chemotherapeutic agents" CANCER BIOTHERAPY AND RADIOPHARMACEUTICALS 2002 US, vol. 17, no. 5, 2002, pages 553-562, XP002505357 ISSN: 1084-9785 * abstract * | 1-11 | |
| Y | TSUNODA A ET AL: "REPEATED ADMINISTRATION OF LOW-DOSE CISPLATIN SIMULTANEOUS WITH URACIL AND TEGAFUR AGAINST AUTOCHTONOUS COLON CANCER IN RATS" SHOWA UNIVERSITY JOURNAL OF MEDICAL SCIENCES, SHOWA UNIVERSITY, TOKYO, JP, vol. 10, no. 1, 1 June 1998 (1998-06-01), pages 41-49, XP000910628 ISSN: 0915-6380 * abstract * * page 47, paragraph 2-5 * | 1-11 | TECHNICAL FIELDS SEARCHED (IPC) A61K A61P |

-/--

The present search report has been drawn up for all claims

| Place of search | Date of completion of the search | Examiner |
|---|---|---|
| Munich | 9 November 2010 | Herrera, Suzanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 17 3256

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GARDANA ET AL: "Analysis of the polyphenolic fraction of propolis from different sources by liquid chromatography-tandem mass spectrometry" JOURNAL OF PHARMACEUTICAL AND BIOMEDICAL ANALYSIS, NEW YORK, NY, US, vol. 45, no. 3, 19 October 2007 (2007-10-19), pages 390-399, XP022306730 ISSN: 0731-7085 * table 2 * ----- | 1-11 | |
| A | ORSOLIC N ET AL: "Synergystic antitumor effect of polyphenolic components of water soluble derivative of propolis against Ehrlich ascites tumour" BIOLOGICAL AND PHARMACEUTICAL BULLETIN 200504 JP, vol. 28, no. 4, April 2005 (2005-04), pages 694-700, XP002505533 * abstract * * page 699, right-hand column * ----- | 1 | |
| A | ORSOLIC N ET AL: "Peroral Application of Water-soluble Derivative of Propolis (WSDP) and Its Related Polyphenolic Compounds and Their Influence on Immunological and Antitumour Activity" VETERINARY RESEARCH COMMUNICATIONS ; AN INTERNATIONAL JOURNAL PUBLISHING TOPICAL REVIEWS AND RESEARCH ARTICLES ON ALL ASPECTS OF THE VETERINARY SCIENCES, KLUWER ACADEMIC PUBLISHERS, DO, vol. 29, no. 7, 1 October 2005 (2005-10-01), pages 575-593, XP019270517 ISSN: 1573-7446 * abstract * * page 576, paragraph 2 * ----- | 1-11 | TECHNICAL FIELDS SEARCHED (IPC) |

The present search report has been drawn up for all claims

| Place of search | Date of completion of the search | Examiner |
|---|---|---|
| Munich | 9 November 2010 | Herrera, Suzanne |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 17 3256

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2010

| Patent document cited in search report | Publication date | Patent family member(s) | Publication date |
|---|---|---|---|
| WO 2010031766 A1 | 25-03-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1168391 A
- US 4328229 A
- US 5008441 A
- WO 03090681 A
- WO 2005105086 A

### Non-patent literature cited in the description

- *Cancer Research,* 1995, vol. 1, 839-845
- **CHIAO, C.** Apoptosis and altered redox state induced by caffeic acid phenethyl ester (CAPE) in transformed rat fibrolast cells. *Cancer Research,* 1995, vol. 55 (3576), 3583
- **GUARINI L ; SU ZZ ; ZUCKER S ; LIN J ; GRUNBERGER D ; FISHER PB.** Growth inhibition and modulation of antigenic phenotype in human melanoma and glioblastoma multiforme cells by caffeic acid phenethyl ester (CAPE. *Division of Pediatric Hematology/ Oncology,* 1991, vol. 4, 231-242
- **MIRZOEVA, O.** The effect of propolis and its components on eicosanoid production during the inflammatory response. *Prostaglandins Leukotienes Essent Fatty Acids,* 441-449
- **ORBAN, Z.** Caffeic acid phenethyl ester induces leukocyte apoptosis, modulates nuclear factor-kappa B and suppresses acute inflammation. *Neuroimmunomodul,* 2000, vol. 7, 99-105
- **NATARAJAN, K.** Caffeic acid phenethyl ester is a potent and specific inhibitor of activation of nuclear transcription factor NF-kappa B. *Immunology,* 1996, vol. 93, 9090-9095
- **MASAHIKO, W. et al.** Caffeic Acid Phenethyl Ester Induces Apoptosis by Inhibition of NF (kappa) B and Activation of Fas in Human Breast Cancer MCF-7 Cells. *Journal of Biological Chemistry,* 2004, vol. 279 (7), 6017-6026
- **TAKEMA, N. et al.** Selective antiproliferative activity of caffeic acid phenethyl ester analogues on highly liver-metastatic murine colon 26-L5 carcinoma cell line. *Bioorganic&Medicinal Chemistry,* 2002, vol. 10 (10), 3351-3359
- *Medical Research Review,* 2003, vol. 23 (4), 519-534
- *Cancer research,* 2001, vol. 61, 118-125